# EUROPEAN PATENT SPECIFICATION

(11) **EP 3 804 783 B1**
(45) Date of publication and mention of the grant of the patent: **02.08.2023**
(21) Application number: 19814954.4
(22) Date of filing: 23.05.2019
(51) Int. Cl.: A61M 5/142, F04B 43/12, F04B 43/00, F04B 43/08, F04B 49/06, F04B 49/10

(54) **CONTROLLER, METHOD FOR CONTROLLING CONTROLLER, LEARNING DEVICE, AND PROGRAM**
STEUERGERÄT, VERFAHREN ZUR STEUERUNG DES STEUERGERÄTS, LERNVORRICHTUNG UND PROGRAMM
DISPOSITIF DE COMMANDE, PROCÉDÉ DE COMMANDE DE DISPOSITIF DE COMMANDE, DISPOSITIF D'APPRENTISSAGE ET PROGRAMME

(30) Priority: 08.06.2018 JP 2018110610; 26.04.2019 JP 2019086497
(43) Date of publication of application: 14.04.2021
(73) Proprietor: OMRON Corporation, Shiokoji-dori, Shimogyo-ku, Kyoto-shi Kyoto 600-8530 (JP)
(72) Inventor: UJITA, Yasuhiro, Kyoto 619-0283 (JP); SHIRAMIZU, Gaku, Kyoto 619-0283 (JP); MATSUYAMA, Takayoshi, Kyoto 619-0283 (JP)
(74) Representative: Becker, Eberhard
(86) International application number: PCT/JP2019/020552
(87) International publication number: WO 2019/235242

(56) References cited:
- JP-A- H09 182 789
- JP-A- 2007 046 564
- JP-A- 2007 270 689
- JP-A- 2007 314 218
- JP-A- 2009 291 659
- JP-A- 2011 053 385
- US-A- 4 256 437
- US-A- 5 395 320
- US-A1- 2012 027 622
- US-A1- 2014 294 610
- US-A1- 2016 106 630
- US-A1- 2018 119 692

## Description

### BACKGROUND

### Technical Field

The present invention relates to a transfusion system with a controller, a method for controlling the controller, and a program.

### Related Art

Conventionally, transfusion devices are known in which a chemical solution in a transfusion tube is transfused by compressing and relaxing the transfusion tube to fill a filling container such as a vial container or the like (for example, see patent literature 1).

In addition, a technique for detecting blockage of the transfusion tube in these transfusion devices is known (for example, see patent literature 2). In the technique described in patent literature 2, whether the transfusion tube is blocked is detected on the basis of a sensitivity of a received signal of ultrasonic waves which are propagated in the chemical solution in the transfusion tube and received by an ultrasonic sensor.

### [Literature of related art]

### [Patent literature]

Patent literature 1: Japanese Patent Laid-open No. 9-262283 (published on October 7th, 1997)
Patent literature 2: Japanese Patent Laid-open No. 2016-214793 (published on December 22nd, 2016)

US 2016/106630 A1 discloses a flow control apparatus adapted for a feeding set having a housing capable of receiving at least a portion of the feeding set, a pumping device for action on the feeding set to produce fluid flow in the feeding set for delivery of fluid. The pumping device has a rotor and a motor operatively connected to the rotor for rotating the rotor to act on the feeding set to produce fluid flow in the feeding set. The apparatus has a sensor arranged with respect to the pumping device to produce a signal indicative of a rotational position of the rotor, and a control circuit in communication with the sensor for receiving the sensor signal from the sensor. Occlusion of a tube can be detected.
US 4 256 437 A discloses a peristaltic infusion pump including a continuous tubular arrangement adapted to pass intravenous fluids therethrough and a peristaltic pump head mounted for rotation about a fixed axis. This pump head includes a plurality of innerconnected, circumferentially spaced tube engaging members adapted to successively engage the tubular arrangement during rotation for peristaltically pumping fluid therethrough. The pump includes a stepping motor which is energized in a particular way for rotating the pump head and which is also used to sense occlusion in the tubular arrangement.
US 2014/294610 A1 discloses a peristaltic pump for pumping a liquid including a flexible tube for guiding the liquid to be pumped, a compression means being actuatable for compressing the flexible tube, an upstream valve means arranged in an upstream direction with respect to the compression means and being actuatable to selectively open or close the flexible tube upstream of the compression means, and a downstream valve means arranged in a downstream direction with respect to the compression means and being actuatable to selectively open or close the flexible tube.

### SUMMARY

### [Problems to be Solved]

Meanwhile, in a transfusion device, a changeover that changes a chemical solution to be transfused by replacing a transfusion tube can be performed. The transfusion tubes having different diameters are used corresponding to products after filling. If a wrong transfusion tube is used to transfuse the chemical solution and perform filling, there is a problem that the product is defective, resulting in product abandonment.

Therefore, it is necessary to confirm whether a wrong transfusion tube is used in a changeover process. As described in the conventional technique, although whether an incorrect transfusion tube is used can also be confirmed using an ultrasonic sensor, there is a concern that the device may be complicated due to separately arranging the ultrasonic sensor. In addition, when the ultrasonic sensor is used, it is necessary to flow the chemical solution in the transfusion tube and detect ultrasonic waves propagating in the chemical solution in the transfusion tube, which complicates the work of the changeover.

An aspect of the present invention is accomplished in view of the situation described above, and an object of the aspect is to achieve a technique capable of efficiently performing correctness/incorrectness determination of the transfusion tube during the changeover.

### [Means to Solve Problems]

In order to solve the problems, the present invention is provided by the appended claims. The following disclosure serves a better understanding of the present invention. A controller has a configuration in which the controller includes a control portion that controls each portion of a transfusion device, wherein the control portion obtains motor shaft information showing an action state of a motor shaft of a driving motor and performs the correctness/incorrectness determination of a transfusion tube on the basis of the motor shaft information, wherein the driving motor drives an actuating portion sequentially compressing and relaxing the transfusion tube of the transfusion device.

In addition, in order to solve the problems, a method for controlling controller according to an aspect of the present disclosure has a configuration in which in the method for controlling the controller which controls each portion of the transfusion device, a driving motor that drives an actuating portion sequentially compressing and relaxing a transfusion tube of the transfusion device is controlled, and correctness/incorrectness determination of the transfusion tube is performed on the basis of motor shaft information of the driving motor.

In addition, in order to solve the problems, a program according to an aspect of the present disclosure has a configuration in which the program enables a computer to function as the controller, and enables the computer to function as the control portion.

In addition, in order to solve the problems, a transfusion system according to an aspect of the present disclosure includes the controller, a driving motor that drives an actuating portion sequentially compressing and relaxing a transfusion tube of the transfusion device, and a motor control portion that controls the drive of the driving motor, wherein the control portion obtains motor shaft information showing an action state of a motor shaft of the driving motor from the motor control portion, and performs correctness/incorrectness determination of the transfusion tube on the basis of the motor shaft information.

### [Effect]

According to the aspect of the present invention, correctness/incorrectness determination of a transfusion tube during changeover can be performed efficiently.

### BRIEF DESCRIPTION OF THE DRAWINGS

FIG. 1 is a diagram showing a schematic structure of a transfusion device according to Embodiment 1.
FIG. 2 is a block diagram showing a main configuration of a controller.
FIG. 3 is a diagram showing an example of motor shaft information.
FIGS. 4 is a diagram showing an example of parameters input into a learning device.
FIG. 5 is a flowchart showing a flow of processing of the learning device.
FIG. 6 is a flowchart showing a flow of correctness/incorrectness determination processing of the controller.
FIG. 7 is a diagram schematically showing a flow of processing of determining a type of a transfusion tube of a controller 50.
FIG. 8 is a diagram showing a relationship among a learned model, a threshold value, and an abnormality degree of each tube.
FIG. 9 is a diagram showing a relationship among the learned model, the threshold value, and the abnormality degree of each tube.
FIG. 10 is a diagram showing a relationship among the learned model, the threshold value, and the abnormality degree of each tube.
FIG. 11 is a diagram showing a schematic structure of a transfusion device according to Embodiment 2.
FIG. 12 is a table showing examples of types of tubes.
FIG. 13 is a diagram schematically showing a pinching clearance.
FIG. 14 shows feature-value scores of other tubes with respect to a tube with an inner diameter of 1 mm.
FIG. 15 shows feature-value scores of other tubes with respect to a tube with an inner diameter of 1.5 mm.
FIG. 16 shows feature-value scores of other tubes with respect to a tube with an inner diameter of 2 mm.
FIG. 17 shows feature-value scores of other tubes with respect to a tube with an inner diameter of 2.5 mm.
FIG. 18 shows feature-value scores of other tubes with respect to a tube with an inner diameter of 3 mm.
FIG. 19 shows feature-value scores of other tubes with respect to a tube with an inner diameter of 4 mm.

### DESCRIPTION OF THE EMBODIMENTS

Embodiments according to an aspect of the present invention (hereinafter, also referred to as "the embodiment") are described below on the basis of drawings.

### §1 Application example

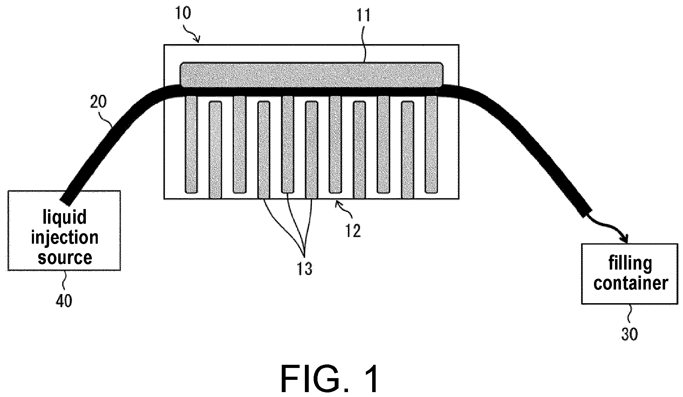

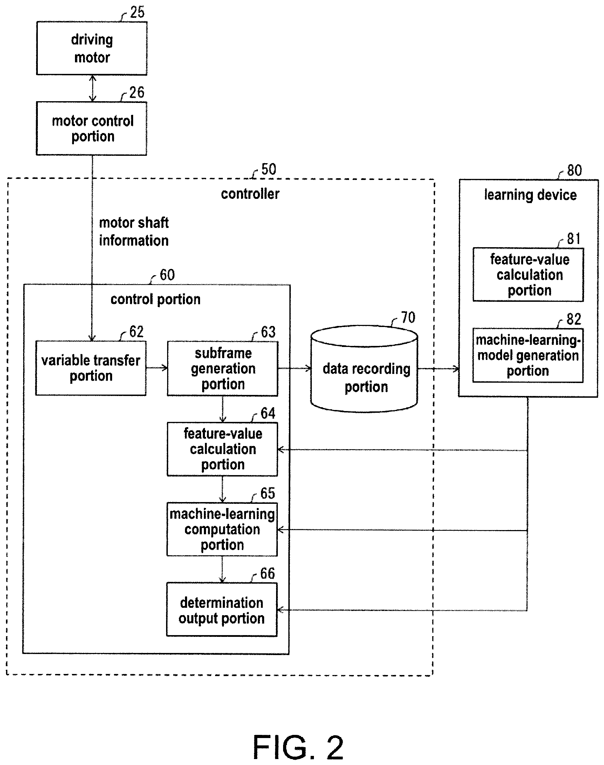

First, an example of a situation in which the present invention is applied is described with reference to FIG. 1 and FIG. 2. FIG. 1 shows an example of a schematic structure of a transfusion device 10 which is controlled by a controller 50 according to the embodiment. FIG. 2 is a block diagram showing a main configuration of the controller 50.

As shown FIG. 1 and FIG. 2, the controller 50 according to the embodiment controls each portion of the transfusion device 10. The transfusion device 10 injects a liquid stored in a liquid injection source 40 into a filling container such as a vial container or the likeby sequentially compressing and relaxing a transfusion tube 20 which extends to the liquid injection source 40 by a plurality of fingers 13. In the transfusion device 10, a changeover that changes the liquid to be transfused by replacing the transfusion tube 20 can be performed.

The transfusion device 10 includes a driving motor 25 that drives the plurality of fingers 13, and a motor control portion 26 that controls rotation of the driving motor 25. The motor control portion 26 supplies motor shaft information including a rotation position, a torque, and a rotation speed of a motor rotating shaft of the driving motor 25 to the controller 50.

The controller 50 is, for example, a programmable logic controller (PLC). The controller 50 includes a control portion 60 as a CPU unit that executes main computation processing. The controller 50 performs correctness/incorrectness determination of the transfusion tube 20 mounted in the transfusion device 10 on the basis of the motor shaft information which is obtained from the motor control portion 26.

In the transfusion device 10, transfusion tubes having different diameters are used for each product to be manufactured using the transfusion device 10. When a wide variety of products is manufactured by the transfusion device 10, it is necessary to replace the transfusion tubes frequently. In the transfusion device 10, when the transfusion tube replacement is performed, the correctness/incorrectness determination of the transfusion tube 20 is performed by driving the driving motor 25 in a state in which the liquid does not enter in the transfusion tube.

The controller 50 may store a threshold value in advance which is statistically generated on the basis of the motor shaft information of a plurality of transfusion tubes 20 in a normal state. Then, the controller 50 may compare the feature value calculated on the basis of the motor shaft information obtained from the motor control portion 26 with the threshold value stored in advance to perform the correctness/incorrectness determination of the transfusion tube 20 mounted in the transfusion device 10.

In addition, the controller 50 may be communicably connected to a learning device 80 that collects the motor shaft information of the plurality of transfusion tubes 20 in the normal state and performs machine learning. Then, the controller 50 may perform the correctness/incorrectness determination of the transfusion tube 20 on the basis of the feature value calculated on the basis of the motor shaft information obtained from the motor control portion 26, and a learned model learned by the learning device 80.

In this way, the controller 50 can perform the correctness/incorrectness determination of the transfusion tube 20 even if the liquid is not injected into the transfusion tube 20 in a changeover process of replacing the transfusion tube 20. In addition, the controller 50 performs the correctness/incorrectness determination of the transfusion tube 20 on the basis of drive shaft information of the driving motor 25 of the transfusion device 10. Therefore, the correctness/incorrectness determination of the transfusion tube 20 can be performed without separately arranging, in the transfusion device 10, a component such as an ultrasonic sensor or the like for detecting the state of the transfusion tube 20.

### §2 Configuration example

A configuration of the controller 50 according to the embodiment of the present invention is specifically described below on the basis of FIGS. 1 to 6.

### [Embodiment 1]

An embodiment of the present invention is specifically described below. FIG. 1 is a diagram schematically showing the example of the schematic structure of the transfusion device 10 which is controlled by the controller 50 according to the embodiment. FIG. 2 is a block diagram showing the main configuration of the controller 50. The controller 50 has a function of performing the correctness/incorrectness determination of the transfusion tube 20 mounted in the transfusion device 10 with reference to the motor shaft information of the driving motor 25 that drives the transfusion device 10.

Moreover, the driving motor 25, the motor control portion 26 that controls drive of the driving motor 25, and the controller 50 are also collectively referred to as a transfusion system.

### (With regard to configuration of transfusion device 10)

As shown in FIG. 1, the transfusion device 10 includes a lid 11 and an actuating portion 12. The lid 11 and the actuating portion 12 are accommodated in an interior of the transfusion device 10. The actuating portion 12 includes a plurality of fingers 13. In addition, the transfusion device 10 is equipped with the replaceable transfusion tube 20. The transfusion tube 20 is pressed against the actuating portion 12 by the lid 11. The transfusion tube 20 is used by being mounted in the transfusion device 10 in a way of extending from the liquid injection source 40 in which the liquid is stored to the filling container 30 such as the vial container or the like which is filled with the liquid.

As shown in FIG. 2, the transfusion device 10 includes the driving motor 25 and the motor control portion 26. The driving motor 25 is a general servomotor. The motor control portion 26 controls a position and a speed of the driving motor 25.

Each of the plurality of fingers 13 is a rod-shaped member that performs linear motion in a direction of approaching the lid 11 or moving away from the lid 11 by the rotation of the driving motor 25 (see FIG. 2). The transfusion tube 20 is sequentially compressed and relaxed between the lid 11 and the fingers 13 along with the motion of each finger 13. In the transfusion device 10, the liquid in the transfusion tube 20 moves from the liquid injection source 40 toward the filling container 30 by this motion of the plurality of fingers 13.

Moreover, in the embodiment, as an example of the transfusion device 10, the configuration of the transfusion device of the so-called finger type is described, in which the transfusion tube 20 is sequentially compressed and relaxed to fill the filling container 30 with the liquid by the linear motion of the plurality of fingers 13 caused by the driving motor 25. However, the transfusion device 10 is not limited to the transfusion device of the finger type as described above, and may be a transfusion device of a peristaltic type that sequentially compresses and relaxes a tube by a rotary roller.

### (With regard to configuration of controller 50)

As shown in FIG. 2, the controller 50 includes the control portion 60 and a data recording portion 70. In addition, the controller 50 is communicably connected to the learning device 80.

The control portion 60 is a computation device having a function of comprehensively controlling each portion of the transfusion device 10. The control portion 60 obtains the motor shaft information which shows an action state of the motor shaft of the driving motor 25 from the motor control portion 26, and supplies a control signal generated on the basis of the obtained motor shaft information to the motor control portion 26, and thereby the control portion 60 can control the drive of the driving motor 25.

The data recording portion 70 is a storage for storing various data used in the controller 50.

### (With regard to configuration of control portion 60)

The control portion 60 includes a variable transfer portion 62, a subframe generation portion 63, a feature-value calculation portion 64, a machine-learning computation portion 65 and a determination output portion 66.

The variable transfer portion 62 obtains, from the motor control portion 26, the motor shaft information showing the action state of the motor shaft including the information of the position, the speed and the torque of the driving motor 25.

The subframe generation portion 63 determines, with reference to the obtained motor shaft information, a subframe for confirming the motion of the transfusion device 10. The subframe generation portion 63 generates the subframe of the motor shaft information in a period in which a drive state of the driving motor 25 is a predetermined state.

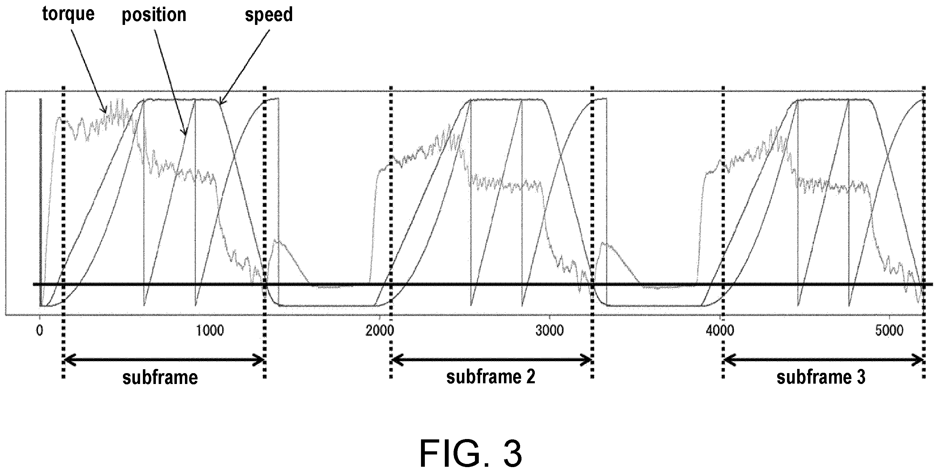

FIG. 3 is a diagram showing an example of the motor shaft information. In the example shown in FIG. 3, the transfusion device 10 has a configuration in which the driving motor 25 is rotated three times to perform one filling. The action of the driving motor 25 is stopped for a pregiven time between the one filling and the next filling. While the action of the driving motor 25 is stopped, there is no change in the position, the speed and the torque of the motor shaft. Therefore, the subframe generation portion 63 sets the period, in which the driving motor 25 drives and the liquid filling is performed, as the subframe in the obtained motor shaft information.

The subframe generation portion 63 performs, for example, subframe generation processing of extracting, as the subframe, a period in which the rotation speed of the motor shaft of the driving motor 25 is equal to or greater than a predetermined value. The subframe generation portion 63 sets, for example, a section in which the rotation speed of the motor shaft of the driving motor 25 is speed > 10 pulse/s as a subframe, and extracts motor shaft information in the subframe. The subframe generation portion 63 supplies the extracted motor shaft information to at least one of the data recording portion 70 and the feature-value calculation portion 64.

The feature-value calculation portion 64 uses the motor shaft information extracted by the subframe generation portion 63 to calculate the feature value that shows a feature of the drive state of the driving motor 25. In this way, the feature-value calculation portion 64 calculates a feature value of the motor shaft in the period in which the drive state of the driving motor 25 is in the predetermined state. For example, the feature-value calculation portion 64 calculates, as the feature value, an average value of the torque in the section in which the rotation speed of the motor shaft of the driving motor 25 is speed > 10 pulse/s. The feature-value calculation portion 64 supplies the calculated feature value to the machine-learning computation portion 65.

The machine-learning computation portion 65 takes the feature value calculated by the feature-value calculation portion 64 as an input, and calculates an abnormality degree with reference to a learned model which is made by a learning flow described later. In the machine-learning computation portion 65, for example, the average value of the torque is input as the feature value, and the abnormality degree of the average value of the torque with respect to the learned model is calculated.

The determination output portion 66 takes the abnormality degree calculated by the machine-learning computation portion 65 as an input, and performs the correctness/incorrectness determination of the transfusion tube 20 corresponding to whether the abnormality degree exceeds the threshold value made by the learning flow described later.

In this way, the controller 50 calculates the feature value in the period in which the drive state of the driving motor 25 is in the predetermined state with reference to the motor shaft information of the driving motor 25, and performs the correctness/incorrectness determination of the transfusion tube 20 on the basis of the feature value. For example, the controller 50 performs the correctness/incorrectness determination of the transfusion tube 20 on the basis of the torque value of the motor shaft of the driving motor in the period in which the rotation speed of the motor shaft of the driving motor 25 is equal to or greater than the predetermined value. Therefore, the controller 50 can efficiently perform the correctness/incorrectness determination of the transfusion tube 20 during the changeover without separately arranging a sensor such as an ultrasonic sensor or the like.

### (With regard to learned model of transfusion tube 20)

In the transfusion device 10, a suitable transfusion tube 20 is selected from the plurality of transfusion tubes 20 having diameters different from each other corresponding to the product to be manufactured, and the suitable transfusion tube 20 is mounted. When the transfusion tubes 20 have different diameters, each value of the motor shaft of the driving motor 25 compressing and relaxing the transfusion tube 20 is different.

The learning device 80 is connected to the controller 50. The learning device 80 obtains, from the controller 50, the information of the speed, the torque, the position, and the like of the motor shaft of the driving motor 25 with regard to each of all the transfusion tubes 20 used in the transfusion device 10. Then, the learning device 80 performs the machine learning with regard to the motor shaft information, and generates the learned model which shows the normal state of the motor shaft information with regard to each transfusion tube 20. The learning device 80 transfers the generated learned model to the controller 50.

The learning device 80 includes a feature-value calculation portion 81 and a machine-learning-model generation portion 82.

The feature-value calculation portion 81 calculates the feature value with reference to the subframe of the value of the motor shaft of the driving motor 25, which is recorded in the data recording portion 70 of the controller 50. Particularly, the feature-value calculation portion 81 calculates the feature value with reference to the subframe of the value of the motor shaft of the driving motor 25 when the transfusion device 10 is operated in a state in which the liquid does not enter the transfusion tube 20.

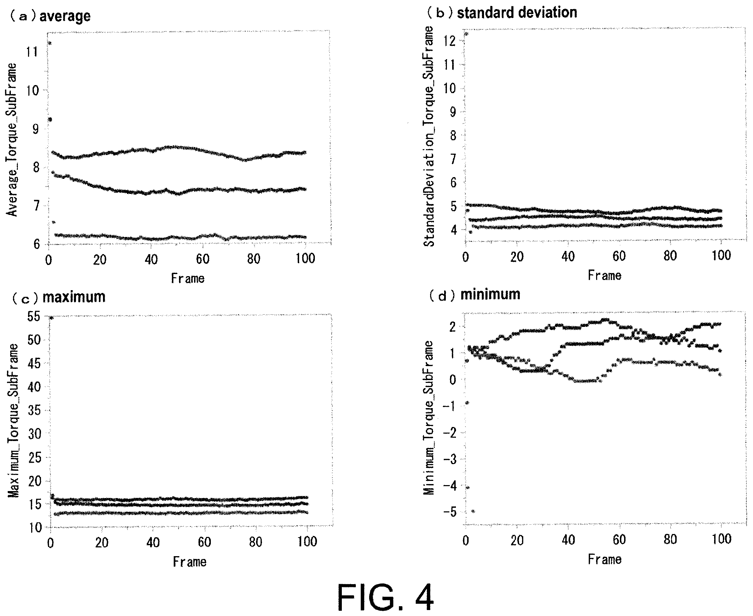

(a) to (d) of FIG. 4 are diagrams showing an example of parameters input into the learning device 80. (a) of FIG. 4 is a diagram showing the average value of the torque of the motor shaft of the driving motor 25. (b) of FIG. 4 is a diagram showing standard deviation of the torque of the motor shaft of the driving motor 25. (c) of FIG. 4 is a diagram showing a maximum value of the torque of the motor shaft of the driving motor 25. (d) of FIG. 4 is a diagram showing a minimum value of the torque of the motor shaft of the driving motor 25. In the learning device 80, the feature-value calculation portion 81 may input a plurality of parameters with regard to the motor shaft of the driving motor 25.

Moreover, although only the parameters according to the torque of the motor shaft of the driving motor 25 are exemplified in FIG. 4, in addition to the torque, the parameters according to the speed or the position also can be input to the learning device 80. The feature-value calculation portion 81 calculates the feature value showing the normal state of each of the transfusion tubes 20 with reference to these input parameters.

The machine-learning-model generation portion 82 performs the machine learning with regard to the feature value of each transfusion tube 20 which is calculated by the feature-value calculation portion 81, and generates the learned model showing the normal state of a correlation between each transfusion tube 20 and the motor shaft information. In addition, the machine-learning-model generation portion 82 generates, on the basis of the learned model, the threshold value of the abnormality degree for separating each transfusion tube 20 from other transfusion tubes 20.

### (With regard to flow of processing of generating learned model)

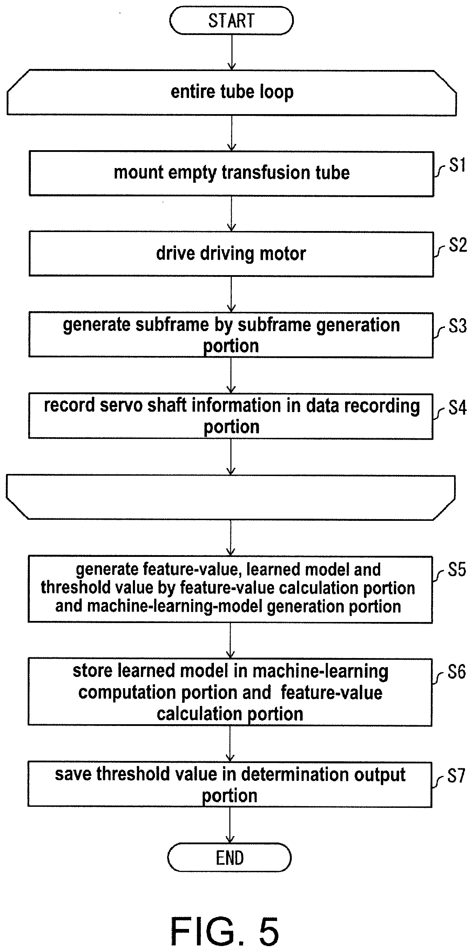

FIG. 5 is a flowchart showing a flow of processing of the learning device 80 and shows the flow of the processing of generating the learned model. For example, in a process of performing calibration of the transfusion device 10, the processing of generating the learned model is executed by the controller 50 and the learning device 80.

When executing the processing of generating the learned model, first, an empty transfusion tube 20 which the liquid does not enter is mounted in the transfusion device 10 (step S1).

Subsequently, the driving motor 25 is driven by the function of the motor control portion 26. The control portion 60 of the controller 50 obtains the motor shaft information by the function of the variable transfer portion 62 (step S2).

The control portion 60 generates the subframe by the function of the subframe generation portion 63 on the basis of the motor shaft information obtained via the variable transfer portion 62 (step S3).

The control portion 60 records, in the data recording portion 70, the motor shaft information in the subframe which is generated by the subframe generation portion 63 (step S4).

The controller 50 repeatedly executes the processing of steps S1 to S4 with regard to all the transfusion tubes 20 that are appropriately replaced and used in the infusion device 10.

The learning device 80 obtains the subframe of the motor shaft information recorded in the data recording portion 70 of the controller 50, and calculates the feature value of each transfusion tube 20 by the function of the feature-value calculation portion 81. In addition, the learning device 80 generates the learned model and the threshold value of the abnormality degree of each transfusion tube 20 by the function of the machine-learning-model generation portion 82 (step S5)
The learning device 80 supplies the learned model of each transfusion tube 20 to the controller 50. The control portion 60 of the controller 50 stores the obtained learned model of each transfusion tube 20 in the feature-value calculation portion 64 and the machine-learning computation portion 65 (step S6)
In addition, the learning device 80 supplies the threshold value of the abnormality degree of each transfusion tube 20 to the controller 50. The control portion 60 of the controller 50 stores the obtained threshold value of the abnormality degree of each transfusion tube 20 in the determination output portion 66 (step S7).

(With regard to correctness/incorrectness determination processing of transfusion tube 20 performed by controller 50)

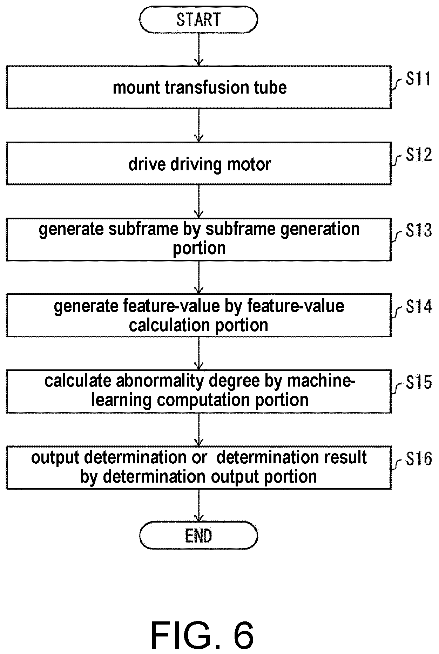

FIG. 6 is a flowchart showing a flow of the correctness/incorrectness determination processing of the transfusion tube 20 performed by the controller 50. The controller 50 performs the correctness/incorrectness determination of determining whether the transfusion tube 20 is incorrectly attached with respect to the product to be manufactured in the changeover process of replacing the transfusion tube 20. The controller 50 performs the correctness/incorrectness determination of the transfusion tube 20 corresponding to whether the abnormality degree of the feature value of the mounted transfusion tube 20 with respect to the learned model, which is generated by the learning device 80 and shows the normal state of the correlation between each transfusion tube 20 and the motor shaft information, exceeds the threshold value.

The mounted transfusion tube 20 is replaced in the transfusion device 10. The empty transfusion tube 20 which the liquid does not enter is mounted in the transfusion device 10 (step S11).

Subsequently, the driving motor 25 is driven by the function of the motor control portion 26. The actuating portion 12 sequentially compresses and relaxes the empty transfusion tube 20 by the drive of the driving motor 25. The control portion 60 of the controller 50 obtains the motor shaft information by the function of the variable transfer portion 62 (step S12).

The control portion 60 generates the subframe by the function of the subframe generation portion 63 on the basis of the motor shaft information obtained via the variable transfer portion 62 (step S13).

The control portion 60 calculates the feature value of the mounted transfusion tube 20 by the function of the feature-value calculation portion 64 with reference to the motor shaft information in the subframe generated by the subframe generation portion 63. The feature-value calculation portion 64 calculates, for example, the average value of the torque of the driving motor 25 in each subframe as the feature value (step S14).

The control portion 60 calculates, by the function of the machine-learning computation portion 65, an abnormality degree of the feature value calculated by the feature-value calculation portion 64 with respect to a learned model of a correct transfusion tube 20 (step S15). Moreover, when the transfusion tube 20 is replaced, a user performs an input operation such as selecting a product to be manufactured and the like on the controller 50. Accordingly, the controller 50 identifies the transfusion tube 20 to be mounted, and uses the learned model of the correct transfusion tube 20 for abnormality degree calculation performed by the machine-learning computation portion 65.

The control portion 60 performs, by the function of the determination output portion 66, the correctness/incorrectness determination of the transfusion tube 20 on the basis of whether the abnormality degree calculated by the machine-learning computation portion 65 exceeds the threshold value of the target transfusion tube 20. Then, the determination output portion 66 outputs a determination result (step S16).

In this way, according to the embodiment, when the transfusion tube 20 of the transfusion device 10 is replaced, the controller 50 obtains the motor shaft information of the driving motor 25, and performs the correctness/incorrectness determination of the transfusion tube 20 on the basis of the motor shaft information. Therefore, in order to perform the correctness/incorrectness determination of the transfusion tube 20, it is not necessary to separately arrange the sensor such as the ultrasonic sensor or the like, and the device and control can be prevented from being complicated.

In addition, the controller 50 uses the learned model, in which the motor shaft information of the driving motor 25 is learned by the machine learning, to calculate the abnormality degree with respect to the learned model, and performs the correctness/incorrectness determination of the transfusion tube 20. In this way, by using the learned model learned by the machine learning, even if the information such as the motor shaft information including a plurality of parameters such as speed, torque, position, and the like is used, the threshold value with respect to a one-dimensional value which is referred to as the abnormality degree may be set to perform the correctness/incorrectness determination. Therefore, the occurrence of defective products due to the incorrect attachment of the transfusion tube 20 can be reliably prevented.

In addition, the controller 50 can perform the correctness/incorrectness determination of the transfusion tube 20 in the state in which the liquid does not enter the transfusion tube 20. Therefore, even if the result of the correctness/incorrectness determination performed by the controller 50 is that the transfusion tube 20 is incorrectly attached, the transfusion tube 20 can be easily replaced again, and the replacement work of the transfusion tube 20 can be efficiently improved.

### [Embodiment 2]

Embodiment 2 of the present invention is described below. Moreover, for convenience of the description, members having the same functions as the members described in the aforementioned Embodiment 1 are designated by the same reference signs, and the description thereof is not repeated. In addition, the configurations of the transfusion device 10, the controller 50, and the learning device 80 according to Embodiment 2 are the same as the configurations of Embodiment 1 described with reference to FIG. 1 and FIG. 2, and the description thereof is omitted.

In Embodiment 1 described above, the aspect is described in which the controller 50 uses the learned model of the normal transfusion tube 20 to perform the correctness/incorrectness determination of the target transfusion tube 20. The controller 50 is not limited to this aspect and can determine the transfusion tube 20 mounted in the transfusion device 10 is which of the plurality of transfusion tubes having different types.

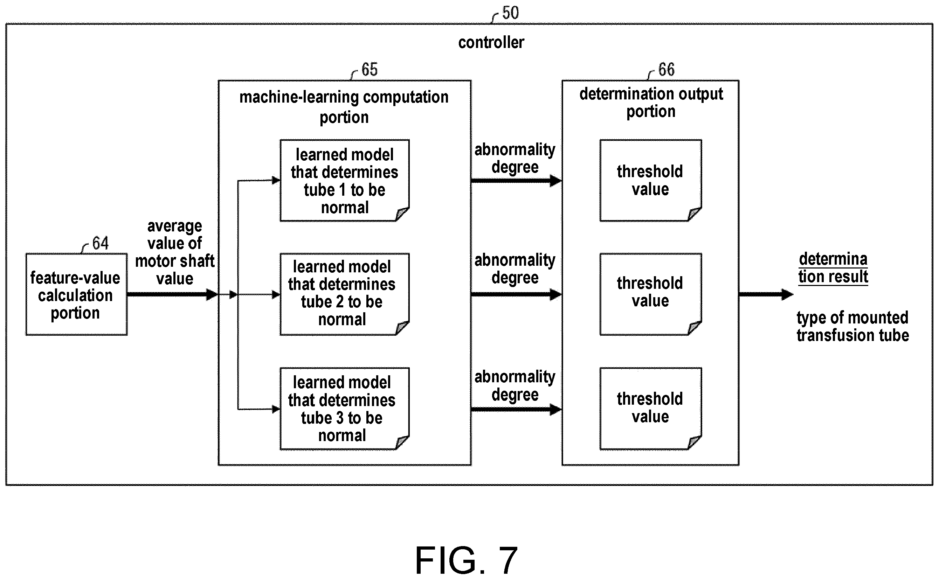

FIG. 7 is a diagram schematically showing a flow of the processing in which the controller 50 determines the type of the transfusion tube 20 mounted in the transfusion device 10. As shown in FIG. 7, the controller 50 calculates, for example, the feature value such as the average value of the torque of the motor shaft of the driving motor 25 or the like by the function of the feature-value calculation portion 64. Subsequently, with regard to the feature value calculated by the feature-value calculation portion 64, the controller 50 calculates, by the function of the machine-learning computation portion 65, the abnormality degree with respect to learned models which determines the transfusion tubes 20 having a plurality of types respectively to be normal.

For example, it is assumed that any of a tube 1, a tube 2, and a tube 3 can be mounted and used in the transfusion device 10. The tube 1, the tube 2, and the tube 3 can be configured as, for example, silicone tubes respectively having diameters of 1.6 mm, 2.4 mm, and 3.2 mm. In this case, the machine-learning computation portion 65 calculates the abnormality degree of the target transfusion tube 20 with respect to learned models which determines the tube 1, the tube 2, and the tube 3 respectively to be normal.

The controller 50 specifies, by the function of the determination output portion 66, the type of the transfusion tube 20 mounted in the transfusion device 10 on the basis of the abnormality degree of the target transfusion tube 20 with respect to a learned model of each tube, and a threshold value of an abnormality degree of each tube.

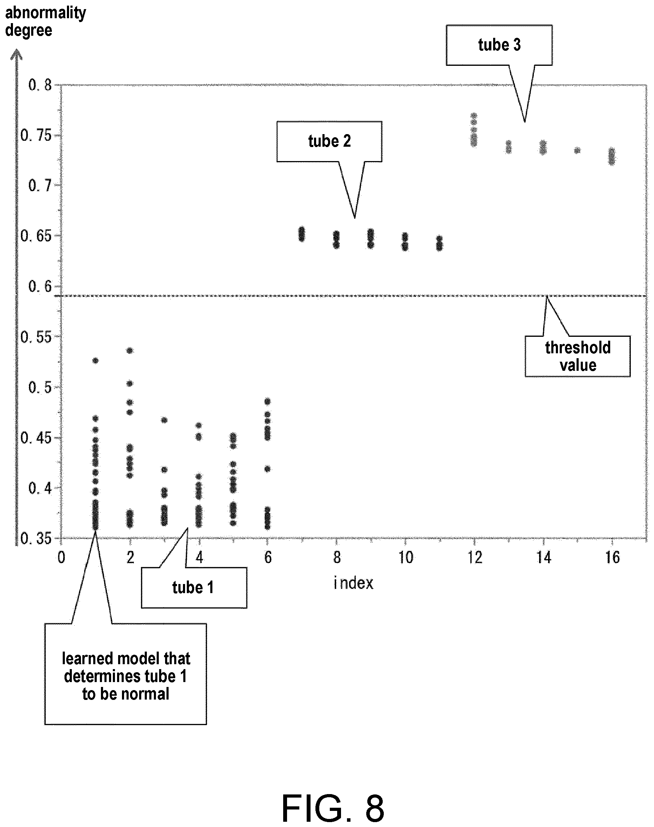

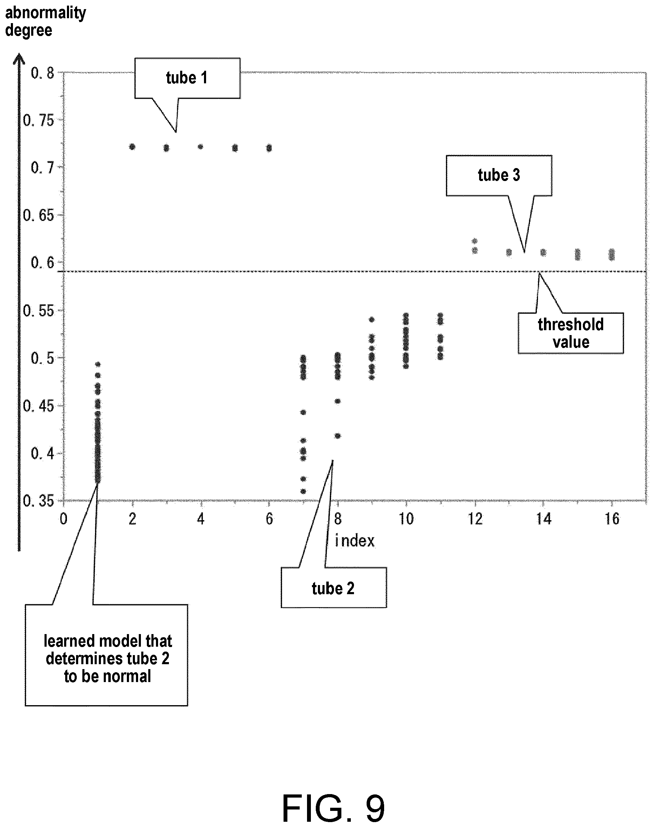

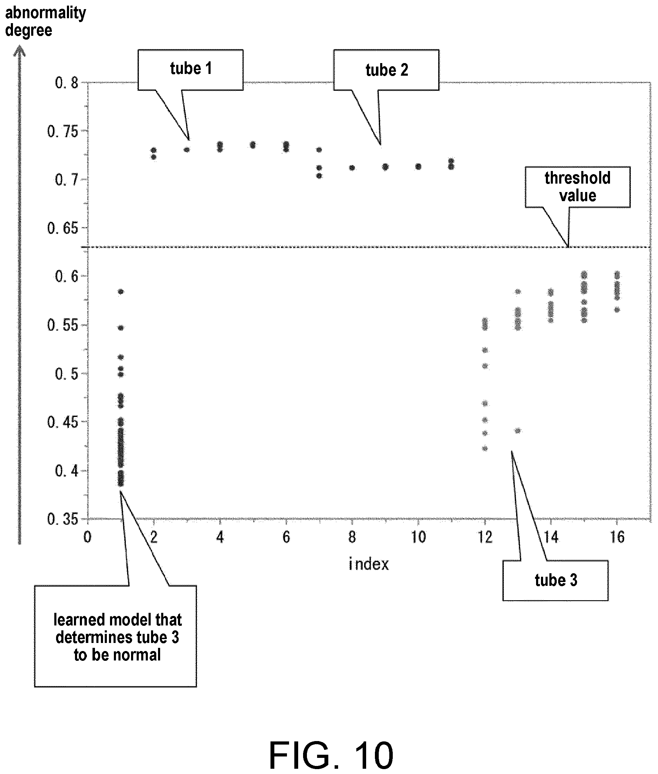

FIG. 8 is a diagram showing a relationship between a learned model which determines the tube 1 to be normal, the threshold value, and the abnormality degree of each tube. FIG. 9 is a diagram showing a relationship between a learned model which determines the tube 2 to be normal, the threshold value, and the abnormality degree of each tube. FIG. 10 is a diagram showing a relationship between a learned model which determines the tube 3 to be normal, the threshold value, and the abnormality degree of each tube.

As shown in FIG. 8, an abnormality degree of a feature value of the tube 1 with respect to the learned model which determines the tube 1 to be normal is equal to or less than the threshold value, and therefore the determination output portion 66 determines that the target transfusion tube is the tube 1. On the other hand, abnormality degrees of feature values of the tube 2 and the tube 3 with respect to the learned model which determines the tube 1 to be normal exceed the threshold value, and therefore the determination output portion 66 determines that the target transfusion tube is neither the tube 2 nor the tube 3.

In addition, as shown in FIG. 9, an abnormality degree of a feature value of the tube 2 with respect to the learned model which determines the tube 2 to be normal is equal to or less than the threshold value, and therefore the determination output portion 66 determines that the target transfusion tube is the tube 2. On the other hand, abnormality degrees of feature values of the tube 1 and the tube 3 with respect to the learned model which determines the tube 2 to be normal exceed the threshold value, and therefore the determination output portion 66 determines that the target transfusion tube is neither the tube 1 nor the tube 3.

In addition, as shown in FIG. 10, an abnormality degree of a feature value of the tube 3 with respect to the learned model which determines the tube 3 to be normal is equal to or less than the threshold value, and therefore the determination output portion 66 determines that the target transfusion tube is the tube 3. On the other hand, abnormality degrees of feature values of the tube 1 and the tube 2 with respect to the learned model which determines the tube 3 to be normal exceed the threshold value, and therefore the determination output portion 66 determines that the target transfusion tube is neither the tube 1 nor the tube 2.

In this way, the controller 50 calculates the feature value of the target transfusion tube 20 on the basis of the motor shaft information of the driving motor 25. Then, with regard to the plurality of transfusion tubes having different types that can be used in the transfusion device 10, the controller 50 calculates the abnormality degree of the feature value of the target transfusion tube 20 with respect to each learned model. The controller 50 can determine the type of the transfusion tube 20 being used with reference to the calculated abnormality degree.

In this way, the controller 50 can identify the mounted transfusion tube 20 with reference to the plurality of learned models stored in the machine-learning computation portion 65 on the basis of the motor shaft information of the driving motor 25. Therefore, in the changeover process of replacing the transfusion tube 20, the user replaces the transfusion tube 20 and executes the identification determination by the controller 50, and thereby whether a correct tube is mounted or an incorrect tube is mounted can be known.

### [Embodiment 3]

Embodiment 3 of the present invention is described below. Moreover, for convenience of the description, members having the same functions as the members described in the aforementioned Embodiment 1 and Embodiment 2 are designated by the same reference signs, and the description thereof is not repeated.

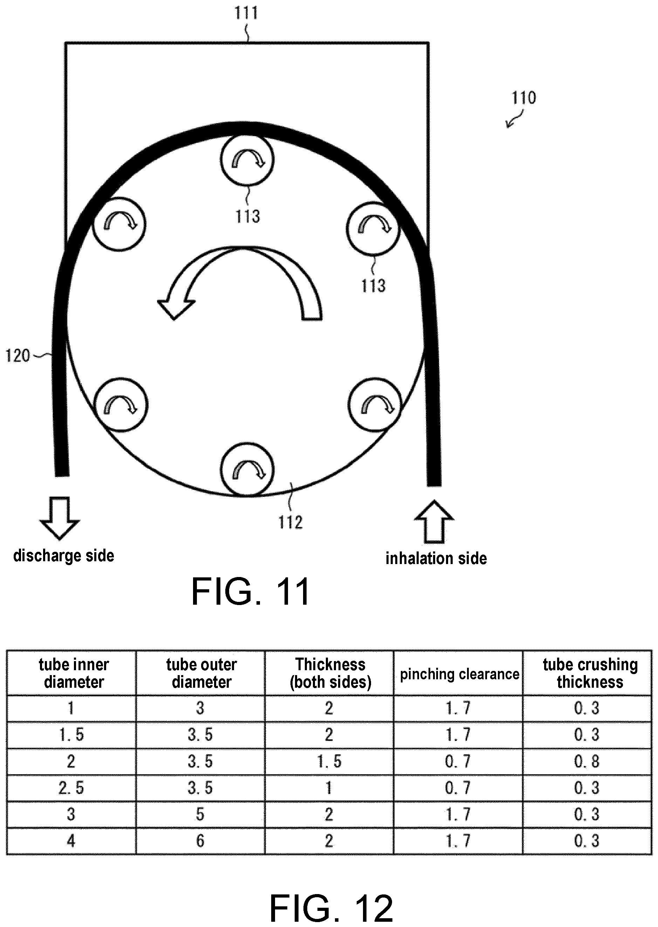

FIG. 11 shows an example of a schematic structure of a transfusion device 110 according to Embodiment 3 which is controlled by the controller 50. A configuration of the controller 50 that controls the transfusion device 110 is the same as the controller 50 described above with reference to FIG. 2, and the description thereof is omitted.

As shown in FIG. 11, in the embodiment, the transfusion device 110 is configured by a peristaltic pump and includes a jig 111, a rotor 112, and rollers 113. The rotor 112 is rotated by the driving motor 25 serving as the servomotor. Moreover, when the driving motor 25 is the servomotor, the controller 50 can obtain the motor shaft information (for example, the torque value) on the basis of control information of the servomotor without arranging the sensor or the like for obtaining the motor shaft information.

The rollers 113 are arranged at predetermined intervals around the rotor 112 and move in a circular motion along with rotation of the rotor 112. In addition, the rollers 113 are arranged in the rotor 112 to rotate freely, and are in contact with a transfusion tube 120 and rotated in a direction opposite to the rotation of the rotor 112.

The transfusion device 110 makes the plurality of rollers 113 rotate by the driving motor 25, and thereby sequentially compresses and relaxes a transfusion tube 120 between the rollers 113 and the jig 111 to send the liquid in the transfusion tube 120 in the same direction as the rotation direction of the rotor 112.

FIG. 12 is a table showing examples of the transfusion tubes 120 used in the transfusion device 110 and attachment states. In the examples showing in FIG. 12, with regard to the transfusion tubes 120, variations of a tube inner diameter, a tube outer diameter, a thickness, a pinching clearance, and a tube crushing thickness are shown. The tube inner diameter shows an inner diameter of the transfusion tube 120, that is, a diameter of a passage through which the liquid flows. The tube outer diameter shows an outer diameter of the transfusion tube 120. The thickness shows a difference between the tube outer diameter and the tube inner diameter, which shows twice a thickness of the tube of the transfusion tube 120, that is, a thickness of the transfusion tube 120 when compressed until the inner diameter of the transfusion tube 120 becomes 0.

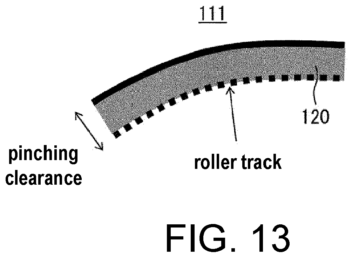

FIG. 13 is a diagram schematically showing the pinching clearance. As shown in FIG. 13, the pinching clearance shows a gap between the rollers 113 and the jig 111. That is, the pinching clearance shows a clearance for pinching and compressing the transfusion tube 120 between the rollers 113 and the jig 111. The pinching clearance is set corresponding to the type of the transfusion tube 120 being used, and the clearance is changed by, for example, replacing the jig 111 or changing a position of the jig 111.

The tube crushing thickness shows an amount of compression of a tube material when the transfusion tube 120 is compressed by the rollers 113. That is, the tube crushing thickness is equivalent to a difference between the thickness and the pinching clearance.

(With regard to correctness/incorrectness determination processing of transfusion tube 120 performed by controller 50)

When the transfusion tube 120 is replaced, the controller 50 performs the correctness/incorrectness determination for determining whether the incorrect attachment of the transfusion tube 120 occurs. After steps S11 to S13 of the flowchart shown in FIG. 6, in step S14, the control portion 60 calculates the feature value with reference to the motor shaft information of the driving motor 25 in each subframe by the function of the feature-value calculation portion 64. In the embodiment, the feature-value calculation portion 64 calculates the feature value on the basis of the torque value of the motor shaft.

As described above, when the driving motor 25 is driven and the transfusion tube 120 is compressed, a repulsive force corresponding to a characteristic of the transfusion tube 120 is generated. Because the repulsive force affects the torque of the motor shaft, information of the characteristic of the transfusion tube 120 is included in the torque value of the motor shaft. Therefore, the feature-value calculation portion 64 can calculate an appropriate feature value corresponding to the characteristic of the transfusion tube 120 by calculating the feature value on the basis of the torque value of the motor shaft.

In step S15, the control portion 60 calculates, by the function of the machine-learning computation portion 65, the abnormality degree (a feature-value score) of the feature value calculated by the feature-value calculation portion 64 with respect to the learned model of the correct transfusion tube 120.

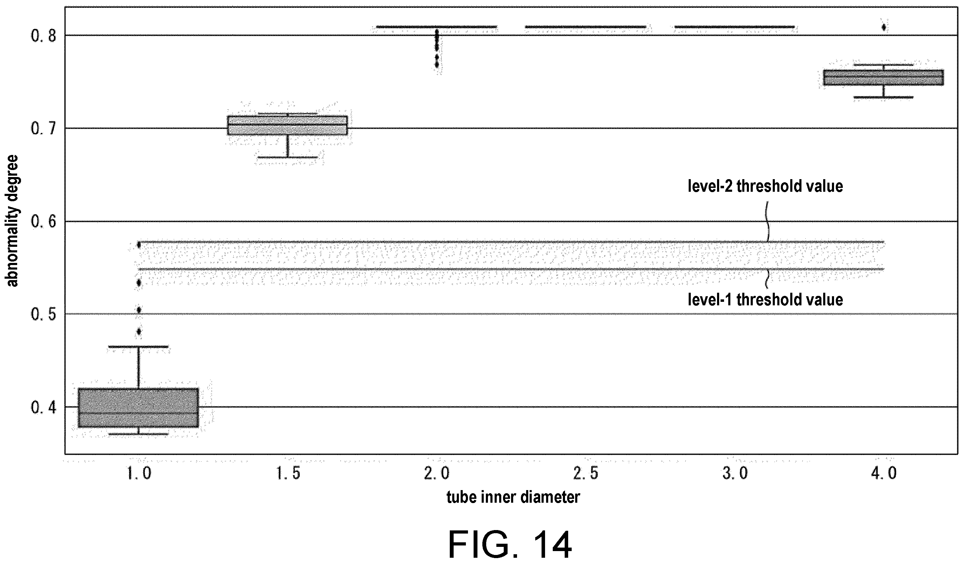

FIGS. 14 to 19 are box plots showing variations of the abnormality degree with respect to the learned model of each transfusion tube 120 when the transfusion tubes 120 having inner diameters of 1 mm, 1.5 mm, 2 mm, 2.5 mm, 3 mm, and 4 mm are respectively determined to be correct. In addition, a horizontal axis shows the inner diameter of the transfusion tube 120, and a vertical axis shows the abnormality degree.

The determination output portion 66 performs the correctness/incorrectness determination of the transfusion tube 120 on the basis of whether the abnormality degree of the target transfusion tube 120 exceeds the predetermined threshold value. Here, the threshold value may be set on the basis of a standard deviation in a distribution of the abnormality degree of the learned model of the correct transfusion tube 120. For example, a value, which is obtained by adding a value obtained by multiplying the standard deviation by a predetermined magnification (for example, 2 to 4 times) to an average value of the distribution of the abnormality degree of the learned model of the correct transfusion tube 120, is set as the threshold value.

FIG. 14 shows variations of abnormality degrees of each transfusion tube 120 when the transfusion tube 120 having an inner diameter of 1 mm is determined to be correct. As shown in FIG. 14, the variation of the abnormality degree of the correct transfusion tube 120 having the inner diameter of 1 mm has a median value less than 0.4 and a maximum value about 0.47. On the other hand, the transfusion tubes 120 having inner diameters of 1.5 mm, 2 mm, 2.5 mm, 3 mm, and 4 mm have the abnormality degrees of about 0.7 to 0.8.

When the target transfusion tube 120 has the inner diameter of 1 mm, the distribution of the abnormality degree does not exceed the threshold value, and therefore the determination output portion 66 determines that a correct transfusion tube 120 is attached. In addition, when the target transfusion tube 120 has an inner diameter of 1.5 mm, 2 mm, 2.5 mm, 3 mm, or 4 mm, the distribution of the abnormality degree exceeds the threshold value, and therefore the determination output portion 66 determines that an incorrect transfusion tube 120 is attached.

The determination output portion 66 can determine whether the abnormality degree of the target transfusion tube 120 exceeds the threshold value with reference to, for example, the threshold value corresponding to required determination precision among the plurality of threshold values such as a level-1 threshold value, a level-2 threshold value, and the like. The determination output portion 66 can perform the correctness/incorrectness determination of the transfusion tube 120 with strict precision by determining the transfusion tube 120 using a smaller threshold value, for example, the level-1 threshold value in the example of FIG. 14. In this case, an incorrect transfusion tube 120 is less likely to be determined to be correct, but a correct transfusion tube 120 is more likely to be determined to be incorrect. On the other hand, when the level-2 threshold value greater than the level-1 threshold value is used, an incorrect transfusion tube 120 is more likely to be determined to be correct, but a correct transfusion tube 120 is less likely to be determined to be incorrect. That is, the determination output portion 66 can change the threshold value according to an instruction of the user corresponding to the required determination precision.

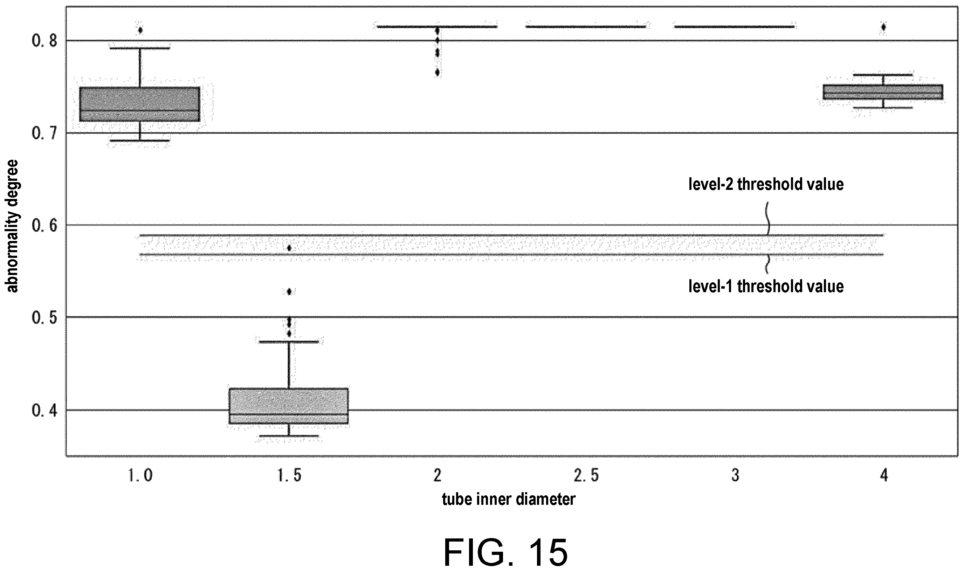

FIG. 15 shows variations of abnormality degrees of each transfusion tube 120 when the transfusion tube 120 having an inner diameter of 1.5 mm is determined to be correct. As shown in FIG. 15, the variation of the abnormality degree of the correct transfusion tube 120 having the inner diameter of 1.5 mm does not exceed the threshold value. On the other hand, the transfusion tubes 120 having inner diameters of 1 mm, 2 mm, 2.5 mm, 3 mm, and 4 mm have the variations of the abnormality degrees exceeding the threshold value.

When the target transfusion tube 120 has the inner diameter of 1.5 mm, the distribution of the abnormality degree does not exceed the threshold value, and therefore the determination output portion 66 determines that a correct transfusion tube 120 is attached. In addition, when the target transfusion tube 120 has an inner diameter of 1 mm, 2 mm, 2.5 mm, 3 mm, or 4 mm, the distribution of the abnormality degree exceeds the threshold value, and therefore the determination output portion 66 determines that an incorrect transfusion tube 120 is attached.

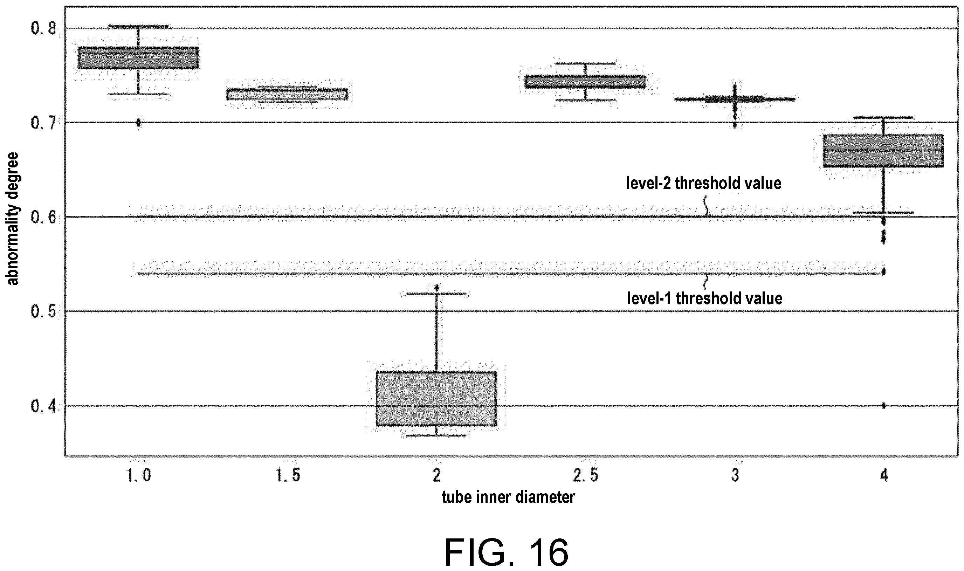

FIG. 16 shows variations of abnormality degrees of each transfusion tube 120 when the transfusion tube 120 having an inner diameter of 2 mm is determined to be correct. As shown in FIG. 16, the variation of the abnormality degree of the correct transfusion tube 120 having the inner diameter of 2 mm does not exceed the threshold value. On the other hand, the transfusion tubes 120 having inner diameters of 1 mm, 1.5 mm, 2.5 mm, 3 mm, and 4 mm have the variations of the abnormality degrees exceeding the threshold value.

When the target transfusion tube 120 has the inner diameter of 2 mm, the distribution of the abnormality degree does not exceed the threshold value, and therefore the determination output portion 66 determines that a correct transfusion tube 120 is attached. In addition, when the target transfusion tube 120 has an inner diameter of 1 mm, 1.5 mm, 2.5 mm, 3 mm, or 4 mm, the distribution of the abnormality degree exceeds the threshold value, and therefore the determination output portion 66 determines that an incorrect transfusion tube 120 is attached.

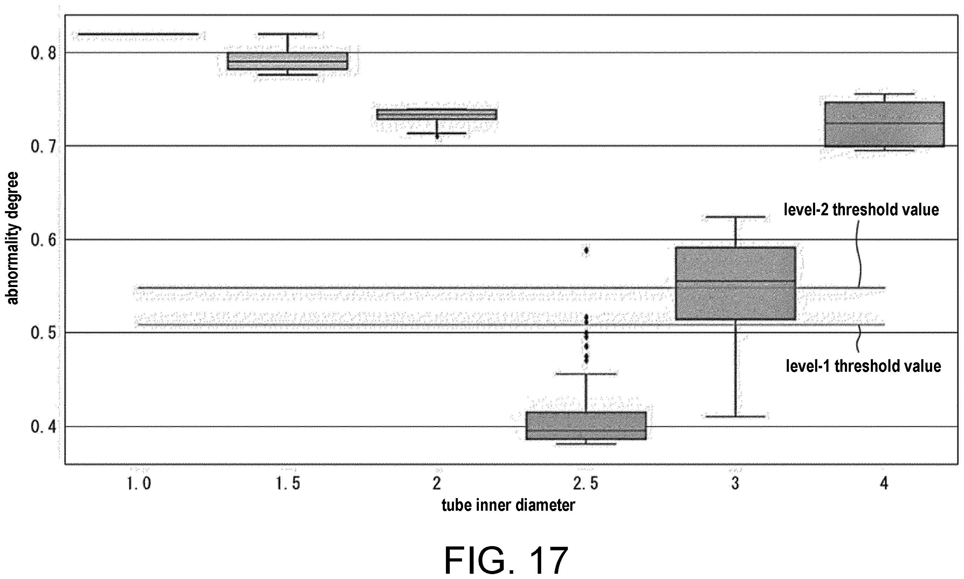

FIG. 17 shows variations of abnormality degrees of each transfusion tube 120 when the transfusion tube 120 having an inner diameter of 2.5 mm is determined to be correct. As shown in FIG. 17, the variation of the abnormality degree of the correct transfusion tube 120 having the inner diameter of 2.5 mm does not exceed the threshold value. On the other hand, the transfusion tubes 120 having inner diameters of 1 mm, 1.5 mm, 2 mm, 3 mm, and 4 mm have the variations of the abnormality degrees exceeding the threshold value.

When the target transfusion tube 120 has the inner diameter of 2.5 mm, the distribution of the abnormality degree does not exceed the threshold value, and therefore the determination output portion 66 determines that a correct transfusion tube 120 is attached. In addition, when the target transfusion tube 120 has an inner diameter of 1 mm, 1.5 mm, 2 mm, 3 mm, or 4 mm, the distribution of the abnormality degree exceeds the threshold value, and therefore the determination output portion 66 determines that an incorrect transfusion tube 120 is attached.

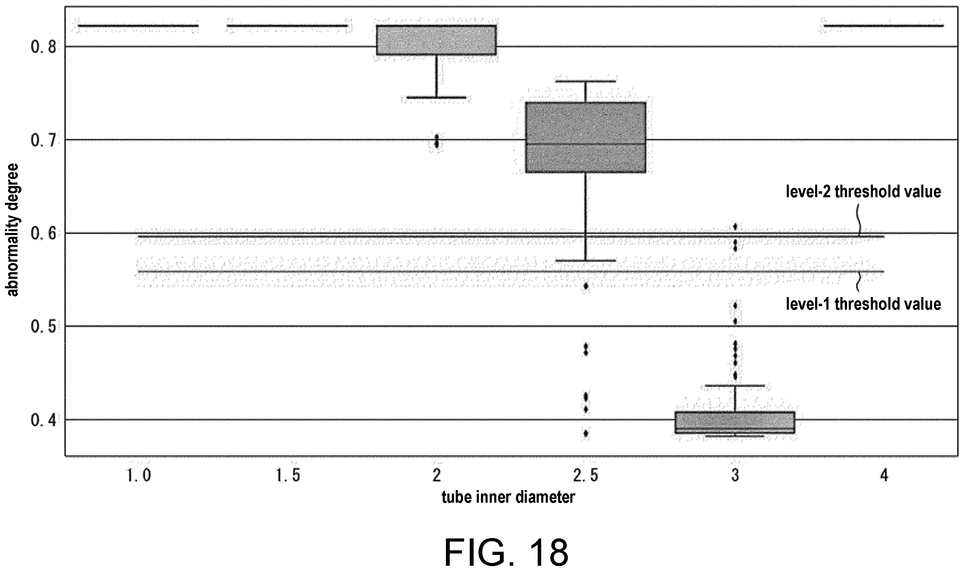

FIG. 18 shows variations of abnormality degrees of each transfusion tube 120 when the transfusion tube 120 having an inner diameter of 3 mm is determined to be correct. As shown in FIG. 18, the variation of the abnormality degree of the correct transfusion tube 120 having the inner diameter of 3 mm does not exceed the threshold value. On the other hand, the transfusion tubes 120 having inner diameters of 1 mm, 1.5 mm, 2 mm, 2.5 mm, and 4 mm have the variations of the abnormality degrees exceeding the threshold value.

When the target transfusion tube 120 has the inner diameter of 3 mm, the distribution of the abnormality degree does not exceed the threshold value, and therefore the determination output portion 66 determines that a correct transfusion tube 120 is attached. In addition, when the target transfusion tube 120 has an inner diameter of 1 mm, 1.5 mm, 2 mm, 2.5 mm, or 4 mm, the distribution of the abnormality degree exceeds the threshold value, and therefore the determination output portion 66 determines that an incorrect transfusion tube 120 is attached.

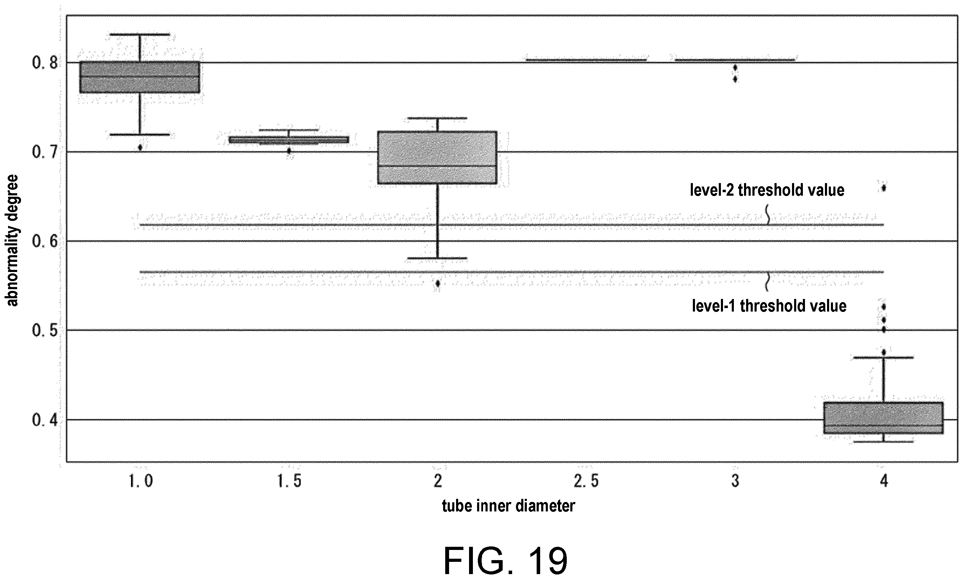

FIG. 19 shows variations of abnormality degrees of each transfusion tube 120 when the transfusion tube 120 having an inner diameter of 4 mm is determined to be correct. As shown in FIG. 19, the variation of the abnormality degree of the correct transfusion tube 120 having the inner diameter of 4 mm does not exceed the threshold value. On the other hand, the transfusion tubes 120 having inner diameters of 1 mm, 1.5 mm, 2 mm, 2.5 mm, and 3 mm have the variations of the abnormality degrees exceeding the threshold value.

When the target transfusion tube 120 has the inner diameter of 4 mm, the distribution of the abnormality degree does not exceed the threshold value, and therefore the determination output portion 66 determines that the correct transfusion tube 120 is attached. In addition, when the target transfusion tube 120 has an inner diameter of 1 mm, 1.5 mm, 2 mm, 2.5 mm, or 3 mm, the distribution of the abnormality degree exceeds the threshold value, and therefore the determination output portion 66 determines that an incorrect transfusion tube 120 is attached.

As described above, the control portion 60 calculates the feature value on the basis of the torque value of the motor shaft and calculates the abnormality degree of the feature value with respect to the learned model of the correct transfusion tube 120 to perform the correctness/incorrectness determination, and thereby a difference of 0.5 mm in the tube diameter can also be properly identified and the correctness/incorrectness determination can be performed. In addition, the correctness/incorrectness determination can be correctly performed even if the inner diameter of the transfusion tube 120 is different and the outer diameter, the thickness, the pinching clearance, and the tube crushing thickness are different, and therefore the correctness/incorrectness determination having robustness can be achieved.

Besides, the control portion 60 can determine the inner diameter of the attached transfusion tube 120 by sequentially performing the correctness/incorrectness determination of each inner diameter.

Moreover, in the examples described above, the examples are shown in which the inner diameters, the outer diameters, the thicknesses, the pinching clearances, and the tube crushing thicknesses of the transfusion tubes 120 are different, and a case in which the materials of the infusion tubes 120 are different can also be dealt with. That is, if the materials of the transfusion tubes 120 are different, flexibilities of the transfusion tubes 120 are different, which leads to differences in the feature-value scores. Therefore, the material of the transfusion tube 120 can also be determined by the correctness/incorrectness determination described above.

Furthermore, when the transfusion tube 120 deteriorates over time, the flexibility of the transfusion tube 120 is different, which leads to the differences in the feature-value scores. Therefore, a degree of the deterioration of the transfusion tube 120 over time can also be determined by the correctness/incorrectness determination described above.

### [Implementation example by software]

A control block of the controller 50 (particularly the subframe generation portion 63, the feature-value calculation portion 64, the machine-learning computation portion 65, and the determination output portion 66), and a control block of the learning device 80 (particularly the feature-value calculation portion 81 and the machine-learning-model generation portion 82) may be achieved by a logic circuit (hardware) formed in an integrated circuit (IC chip) or the like, or may be achieved by software.

In the latter case, the controller 50 and the learning device 80 include a computer which executes a program instruction and is the software that achieves each function. The computer includes, for example, one or more processors and a computer-readable recording medium that stores the program. Besides, in the computer, the processor reads and executes the program from the recording medium, and thereby the object of the present invention is achieved. As the processor, for example, a central processing unit (CPU) can be used. As the recording medium, a "non-temporary tangible medium", for example, in addition to read only memory (ROM) and the like, a tape, a disk, a card, a semiconductor memory, a programmable logic circuit, or the like can be used. In addition, a random access memory (RAM) and the like for expanding the program may be further included. In addition, the program may be supplied to the computer via any transmission medium (a communication network, a broadcast wave, or the like) capable of transmitting the program. Moreover, the aspect of the present invention can be achieved even in a form of a data signal embedded in a carrier wave, in which the program is embodied by electronic transmission.

Moreover, the present invention is not limited to each embodiment described above, and various modifications can be made within a scope shown in claims, and embodiments obtained by appropriately combining technical means respectively disclosed in the different embodiments are also included in the technical scope of the present invention.

In addition, the specific configuration of classification and learning processing for generating the learned model by the learning device 80 is not limited to the present embodiment, and for example, any one of following machine learning methods or a combination thereof can be used.

• Support Vector Machine (SVM)
- Clustering
- Inductive Logic Programming (ILP)
- Genetic Algorithm (GP: Genetic Programming)
- Bayesian Network (BN)
- Neural Network (NN)

When the neural network is used, 3D data may be processed in advance to be used for input of the neural network. In this process, in addition to one-dimensional arrangement or multidimensional arrangement of the data, for example, a method such as Data Argumentation or the like can be used.

In addition, when the neural network is used, a convolutional neural network (CNN) including convolution processing can be used. More specifically, convolution layers for performing a convolution computation may be arranged as one or more layers included in the neural network, and a filter computation (product-sum computation) may be performed on the input date which is input to the layer. In addition, when the filter computation is performed, processing such as padding and the like may be used in combination, or a stride width which is appropriately set may be used.

In addition, as the neural network, a multi-layered or super-multilayered neural network that has the layers ranging from several tens to several thousands may be used.

In addition, a machine learning method which is used in the generation processing of the learned model performed by the learning device 80 may be supervised learning or unsupervised learning.

### [Summarization]

The controller according to the aspect of the present invention includes the control portion that controls each portion of the transfusion device, wherein the control portion obtains the motor shaft information showing the action state of the motor shaft of the driving motor that drives the actuating portion sequentially compressing and relaxing the transfusion tube of the transfusion device, and performs the correctness/incorrectness determination of the transfusion tube on the basis of the motor shaft information.

According to the configuration, the correctness/incorrectness determination of the transfusion tube is performed on the basis of the motor shaft information of the driving motor, and therefore it is not necessary to separately arrange a component such as a sensor or the like for detecting the state of the transfusion tube. In addition, because the correctness/incorrectness determination of the transfusion tube can be performed even if the liquid does not enter the transfusion tube, the transfusion tube after the correctness/incorrectness determination can be replaced easily even if the transfusion tube is incorrectly attached. Therefore, the correctness/incorrectness determination of the transfusion tube during the changeover can be efficiently performed.

In addition, the controller according to the aspect of the present invention has the configuration in which the control portion calculates, with reference to the motor shaft information, the feature value in the period in which the drive state of the driving motor is in the predetermined state, and performs the correctness/incorrectness determination of the transfusion tube on the basis of the feature value.

According to the configuration, the correctness/incorrectness determination of the transfusion tube is performed on the basis of the feature value in the period in which the driving motor is in the drive state. The motor shaft information does not change while the action of the driving motor is stopped, and therefore incorrect correctness/incorrectness determination can be prevented by referring the motor shaft information while the action of the driving motor is stopped.

In addition, the controller according to the aspect of the present invention has the configuration in which the control portion performs, with reference to the motor shaft information, the correctness/incorrectness determination of the transfusion tube on the basis of the torque value of the motor shaft of the driving motor in the period in which the rotation speed of the motor shaft of the driving motor is equal to or greater than the predetermined value.

According to the configuration, the period in which the rotation speed of the motor shaft of the driving motor is equal to or greater than the predetermined value is defined as the period in which the driving motor is acting, and the correctness/incorrectness determination of the transfusion tube is performed on the basis of the torque value of the motor shaft of the driving motor in this period. The torque of the driving motor when the transfusion tube is compressed and relaxed depends on the diameter of the transfusion tube. Therefore, the correctness/incorrectness determination of the transfusion tube can be correctly and efficiently performed by performing the correctness/incorrectness determination of the transfusion tube on the basis of the torque value of the motor shaft of the driving motor in the period in which the driving motor is acting.

In addition, the controller according to the aspect of the present invention has the configuration in which the control portion performs the correctness/incorrectness determination of the transfusion tube by using the learned model in which the motor shaft information of the driving motor is learned by machine learning.

According to the configuration, the plurality of parameters such as the speed, the torque, the position, and the like of the motor shaft of the driving motor are learned by the machine learning to generate the learned model, and the correctness/incorrectness determination of the transfusion tube is performed. When the correctness/incorrectness determination of the transfusion tube is performed without using the machine learning, it is necessary to set an individual threshold value with respect to each parameter to perform the correctness/incorrectness determination. On the other hand, when the correctness/incorrectness determination is performed using the learned model generated by thte machine learning, the one-dimensional threshold value may be set to perform the correctness/incorrectness determination even when the plurality of parameters are used, and the correctness/incorrectness determination can be executed with a high degree of freedom in selection of the parameters.

In addition, the controller according to the aspect of the present invention has the configuration in which the plurality of transfusion tubes having different types can be used in the transfusion device, and the control portion determines the type of the transfusion tube being used on the basis of the motor shaft information of the driving motor.

According to the configuration, which transfusion tube is mounted among the plurality of transfusion tubes having different types that can be used in the transfusion device can be efficiently determined.

In addition, the controller according to the aspect of the present invention has the configuration in which the control portion determines the diameter of the transfusion tube being used on the basis of the motor shaft information of the driving motor.

According to the configuration, which diameter of the transfusion tube is mounted among the plurality of transfusion tubes having different diameters that can be used in the transfusion device can be appropriately determined.

In addition, the controller according to the aspect of the present invention can also be applied to the case in which the peristaltic pump is used as the transfusion device.

In addition, in order to solve the above problems, the method for controlling controller according to the aspect of the present invention has the configuration in which the controller controls each portion of the transfusion device, the driving motor that drives the actuating portion sequentially compressing and relaxing the transfusion tube of the transfusion device is controlled, and the correctness/incorrectness determination of the transfusion tube is performed on the basis of the motor shaft information of the driving motor.

According to the configuration, the correctness/incorrectness determination of the transfusion tube can be performed without separately arranging the component such as the sensor or the like for detecting the state of the transfusion tube. In addition, the correctness/incorrectness determination of the transfusion tube can be performed even if the liquid does not enter the transfusion tube, and therefore the transfusion tube after the correctness/incorrectness determination can be replaced easily even if the transfusion tube is incorrectly attached. Therefore, the correctness/incorrectness determination of the transfusion tube during the changeover can be efficiently performed.

In addition, in order to solve the above problems, the learning device according to the aspect of the present invention obtains the motor shaft information from the controller, generates the learned model of the motor shaft information for the transfusion tube by machine learning, and transfers the generated learned model to the controller.

According to the configuration, the correctness/incorrectness determination of the transfusion tube can be performed using the learned model of the motor shaft information generated by the learning device. Therefore, the correctness/incorrectness determination can be performed using the motor shaft information including the plurality of parameters such as the speed, the position, the torque, and the like and setting the one-dimensional threshold value.

In addition, in order to solve the above problems, the program according to the aspect of the present invention enables the computer to function as the controller, and enables the computer to function as the control portion.

According to the configuration, the computer can function as the controller to perform the correctness/incorrectness determination of the transfusion tube.

In addition, in order to solve the above problems, the program according to the aspect of the present invention enables the computer to function as the learning device.

According to the configuration, the computer can function as the learning device to generate the learned model and perform the correctness/incorrectness determination of the transfusion tube.

In addition, in order to solve the above problems, the transfusion system according to the aspect of the present invention includes: the controller; the driving motor that drives the actuating portion sequentially compressing and relaxing the transfusion tube of the transfusion device; and the motor control portion that controls the drive of the driving motor, wherein the control portion obtains the motor shaft information showing the action state of the motor shaft of the driving motor, and performs the correctness/incorrectness determination of the transfusion tube on the basis of the motor shaft information.

According to the configuration, the correctness/incorrectness determination of the transfusion tube is performed on the basis of the motor shaft information of the driving motor, and therefore it is not necessary to separately arrange the component such as the sensor or the like for detecting the state of the transfusion tube. In addition, because the correctness/incorrectness determination of the transfusion tube can be performed even if the liquid does not enter the transfusion tube, the transfusion tube after the correctness/incorrectness determination can be replaced easily even if the transfusion tube is incorrectly attached. Therefore, the correctness/incorrectness determination of the transfusion tube during the changeover can be efficiently performed.

### [Reference Signs List]

- 10, 110: transfusion device

- 11: lid
- 12: actuating portion
- 13: fingers
- 20, 120: transfusion tube
- 25: driving motor
- 26: motor control portion
- 30: filling container
- 40: liquid injection source
- 50: controller
- 60: control portion
- 63: subframe generation portion
- 64, 81: feature-value calculation portion
- 65: machine-learning computation portion
- 66: determination output portion
- 70: data recording portion
- 80: learning device
- 81: feature-value calculation portion
- 82: machine-learning-model generation portion
- 111: jig
- 112: rotor
- 113: rollers
- S1-S7: steps
- S11-S16: steps

## Claims

1. A transfusion system, comprising:
controller (50), comprising a control portion (60) that controls a transfusion device (10, 110);
a driving motor that drives an actuating portion (12) sequentially compressing and relaxing a transfusion tube (20, 120) of the transfusion device (10, 110); and
a motor control portion (26) that controls the drive of the driving motor (25), wherein, wherein
the control portion (60) obtains motor shaft information showing an action state of a motor shaft of the driving motor (25) from the motor control portion (26), and performs correctness/incorrectness determination for determining whether an incorrect attachment of the transfusion tube (20, 120) occurs on the basis of the motor shaft information,
wherein the motor shaft information comprises a torque value of the motor shaft,
the transfusion system comprising:
a subframe generation portion (63) that generates a subframe of the motor shaft information in a period in which a drive state of the driving motor (25) is in a predetermined state, wherein the predetermined state comprises a rotation speed of the motor shaft of the driving motor (25) is equal to or greater than a predetermined value; and
a feature-value calculation portion (64) that uses the subframe of the motor shaft information generated by the subframe generation portion (63) to calculate, as a feature value that shows a feature of the drive state of the driving motor (25), an average value of a torque in a section of the subframe,
the control portion (60) performs the correctness/incorrectness determination of the transfusion tube (20, 120) on the basis of the feature value.

2. The transfusion system according to claim 1, wherein the control portion (60) performs,
with reference to the motor shaft information,
the correctness/incorrectness determination of the transfusion tube (20, 120) on the basis of the torque value of the motor shaft of the driving motor (25) in a period in which the rotation speed of the motor shaft of the driving motor (25) is equal to or greater than the predetermined value.

3. The transfusion system according to any one of claim 1 or 2, wherein
the control portion (60) performs the correctness/incorrectness determination of the transfusion tube (20, 120) by using a learned model in which the motor shaft information of the driving motor (25) is learned by machine learning.

4. The transfusion system according to any one of claims 1 to 3, wherein
a plurality of the transfusion tubes (20, 120) having different types are used in the transfusion device, and
the control portion (60) determines a type of the transfusion tube (20, 120) being used on the basis of the motor shaft information of the driving motor.

5. The transfusion system according to claim 4, wherein
the control portion (60) determines a diameter of the transfusion tube (20, 120) being used.

6. The transfusion system according to any one of claims 1 to 5, wherein the transfusion device (10, 110) is a peristaltic pump.

7. A method for controlling a controller (50) that controls a transfusion device (10, 110), wherein
a driving motor (25) that drives an actuating portion (12) sequentially compressing and relaxing a transfusion tube (20, 120) of the transfusion device (10, 110) is controlled, and
correctness/incorrectness determination for determining whether an incorrect attachment of the transfusion tube (20, 120) occurs is performed on the basis of motor shaft information of the driving motor (25),
wherein the motor shaft information comprises a torque value of a motor shaft,
the method being **characterized in** further comprising
generating a subframe of the motor shaft information in a period in which a drive state of the driving motor (25) is in a predetermined state, wherein the predetermined state comprises a rotation speed of the motor shaft of the driving motor (25) is equal to or greater than a predetermined value;
using the generated subframe of the motor shaft information to calculate, as a feature value that shows a feature of the drive state of the driving motor (25), an average value of a torque in a section of the subframe; and
performing the correctness/incorrectness determination of the transfusion tube (20, 120) on the basis of the feature value.

8. A computer program comprising instructions which, when the program is executed by a computer, cause the computer to carry out the method of claim 7.

## Patentansprüche

1. Transfusionssystem, umfassend:
eine Steuerung (50), die einen Steuerabschnitt (60) umfasst, der eine Transfusionsvorrichtung (10, 110) steuert;
einen Antriebsmotor, der einen Betätigungsabschnitt (12) antreibt, der einen Transfusionsschlauch (20, 120) der Transfusionsvorrichtung (10, 110) sequentiell komprimiert und entspannt; und
einen Motorsteuerabschnitt (26), der den Antrieb des Antriebsmotors (25) steuert, wobei
der Steuerabschnitt (60) Motorwelleninformationen, die einen Aktionszustand einer Motorwelle des Antriebsmotors (25) zeigen, von dem Motorsteuerabschnitt (26) erhält und basierend auf den Motorwelleninformationen eine Korrektheits-/ Fehlerhaftigkeitsbestimmung durchführt, um zu bestimmen, ob eine fehlerhafte Befestigung des Transfusionsschlauchs (20, 120) auftritt,
wobei die Motorwelleninformationen einen Drehmomentwert der Motorwelle umfassen,
wobei das Transfusionssystem umfasst:
einen Unterrahmen-Erzeugungsabschnitt (63), der einen Unterrahmen der Motorwelleninformationen in einer Periode erzeugt, in der ein Antriebszustand des Antriebsmotors (25) in einem vorbestimmten Zustand ist, wobei der vorbestimmte Zustand eine Drehgeschwindigkeit der Motorwelle des Antriebsmotors (25) umfasst, die gleich oder größer als ein vorbestimmter Wert ist; und
einen Merkmalswert-Berechnungsabschnitt (64), der den Unterrahmen der Motorwelleninformationen, erzeugt von dem Unterrahmen-Erzeugungsabschnitt (63) verwendet, um als einen Merkmalswert, der ein Merkmal des Antriebszustands des Antriebsmotors (25) zeigt, einen Durchschnittswert eines Drehmoments in einem Abschnitt des Unterrahmens zu berechnen,
das Steuerteil (60) die Korrektheit-/Fehlerhaftigkeitsbestimmung des Transfusionsschlauchs (20, 120) basierend auf dem Merkmalswert durchführt.

2. Transfusionssystem gemäß Anspruch 1, wobei der Steuerabschnitt (60),
unter Bezugnahme auf die Motorwelleninformationen,
die Korrektheit-/Fehlerhaftigkeitsbestimmung des Transfusionsschlauchs (20, 120) basierend auf dem Drehmomentwert der Motorwelle des Antriebsmotors (25) in einer Periode durchführt, in der die Drehgeschwindigkeit der Motorwelle des Antriebsmotors (25) gleich oder größer als der vorbestimmte Wert ist.

3. Transfusionssystem gemäß einem der Ansprüche 1 oder 2, wobei
der Steuerabschnitt (60) die Korrektheit-/Fehlerhaftigkeitsbestimmung des Transfusionsschlauchs (20, 120) unter Verwendung eines gelernten Modells durchführt, bei dem die Motorwelleninformationen des Antriebsmotors (25) durch maschinelles Lernen gelernt wird.

4. Transfusionssystem gemäß einem der Ansprüche 1 bis 3, wobei
eine Vielzahl von Transfusionsschläuchen (20, 120) mit unterschiedlichen Typen, in der Transfusionsvorrichtung verwendet werden und
der Steuerabschnitt (60) einen Typ des verwendeten Transfusionsschlauchs (20, 120) basierend auf den Motorwelleninformationen des Antriebsmotors bestimmt.

5. Transfusionssystem gemäß Anspruch 4, wobei
der Steuerabschnitt (60) einen Durchmesser des verwendeten Transfusionsschlauchs (20, 120) bestimmt.

6. Transfusionssystem gemäß einem der Ansprüche 1 bis 5, wobei die Transfusionsvorrichtung (10, 110) eine Peristaltikpumpe ist.

7. Verfahren zur Steuerung eines Controllers (50), der eine Transfusionsvorrichtung (10, 110) steuert, wobei
ein Antriebsmotor (25), der einen Betätigungsabschnitt (12) antreibt, der einen Transfusionsschlauch (20, 120) der Transfusionsvorrichtung (10, 110) sequentiell komprimiert und entspannt, gesteuert wird, und
eine Korrektheit-/Fehlerhaftigkeitsbestimmung zum Bestimmen, ob eine fehlerhafte Befestigung des Transfusionsschlauchs (20, 120) auftritt, basierend auf den Motorwelleninformationen des Antriebsmotors (25) durchgeführt wird,
wobei die Motorwelleninformationen einen Drehmomentwert einer Motorwelle umfassen,
wobei das Verfahren **dadurch gekennzeichnet ist, dass** es ferner umfasst
Erzeugen eines Unterrahmens der Motorwelleninformationen in einer Periode, in der ein Antriebszustand des Antriebsmotors (25) in einem vorbestimmten Zustand ist, wobei der vorbestimmte Zustand eine Drehgeschwindigkeit der Motorwelle des Antriebsmotors (25) umfasst, die gleich oder größer als ein vorbestimmter Wert ist;
Verwenden des erzeugten Unterrahmens der Motorwelleninformationen, um als einen Merkmalswert, der ein Merkmal des Antriebszustandes des Antriebsmotors (25) zeigt, einen Durchschnittswert eines Drehmoments in einem Abschnitt des Unterrahmens zu berechnen; und
Durchführen der Korrektheit-/Fehlerhaftigkeitsbestimmung des Transfusionsschlauchs (20, 120) basierend auf dem Merkmalswert.

8. Computerprogram, umfassend Anweisungen, die, wenn das Programm von einem Computer ausgeführt wird, den Computer veranlassen das Verfahren gemäß Anspruch 7 auszuführen.

## Revendications

1. Système de transfusion, comprenant :
un dispositif de commande (50), comprenant une partie de commande (60) qui commande un dispositif de transfusion (10, 110) ;
un moteur d'entraînement qui entraîne une partie d'actionnement (12) compressant et relâchant séquentiellement un tube de transfusion (20, 120) du dispositif de transfusion (10, 110) ; et
une partie de commande de moteur (26) qui commande l'entraînement du moteur d'entraînement (25), dans lequel
la partie de commande (60) obtient des informations d'arbre de moteur indiquant un état d'action d'un arbre de moteur du moteur d'entraînement (25) depuis la partie de commande de moteur (26), et réalise une détermination d'exactitude/inexactitude pour déterminer si un raccordement incorrect du tube de transfusion (20, 120) survient sur la base des informations d'arbre de moteur,
dans lequel les informations d'arbre de moteur comprennent une valeur de couple de l'arbre de moteur, le système de transfusion comprenant :
une partie de génération de sous-trame (63) qui génère une sous-trame des informations d'arbre de moteur dans une période au cours de laquelle un état d'entraînement du moteur d'entraînement (25) est dans un état prédéterminé, dans lequel l'état prédéterminé comprend qu'une vitesse de rotation de l'arbre de moteur du moteur d'entraînement (25) est supérieure ou égale à une valeur prédéterminée ; et
une partie de calcul de valeur de caractéristique (64) qui utilise la sous-trame des informations d'arbre de moteur générées par la partie de génération de sous-trame (63) pour calculer, en tant qu'une valeur de caractéristique qui indique une caractéristique de l'état d'entraînement du moteur d'entraînement (25), une valeur moyenne d'un couple dans une section de la sous-trame,
la partie de commande (60) réalise la détermination d'exactitude/inexactitude du tube de transfusion (20, 120) sur la base de la valeur de caractéristique.

2. Système de transfusion selon la revendication 1, dans lequel la partie de commande (60) réalise,
en référence aux informations d'arbre de moteur,
la détermination d'exactitude-inexactitude du tube de transfusion (20, 120) sur la base de la valeur de couple de l'arbre de moteur du moteur d'entraînement (25) dans une période au cours de laquelle la vitesse de rotation de l'arbre de moteur du moteur d'entraînement (25) est supérieure ou égale à la valeur prédéterminée.

3. Système de transfusion selon la revendication 1 ou 2, dans lequel
la partie de commande (60) réalise la détermination d'exactitude/inexactitude du tube de transfusion (20, 120) en utilisant un modèle appris dans lequel les informations d'arbre de moteur du moteur d'entraînement (25) sont apprises par apprentissage automatique.

4. Système de transfusion selon l'une quelconque des revendications 1 à 3, dans lequel
une pluralité des tubes de transfusion (20, 120) ayant des types différents sont utilisés dans le dispositif de transfusion, et
la partie de commande (60) détermine un type du tube de transfusion (20, 120) qui est utilisé sur la base des informations d'arbre de moteur du moteur d'entraînement.

5. Système de transfusion selon la revendication 4, dans lequel
la partie de commande (60) détermine un diamètre du tube de transfusion (20, 120) qui est utilisé.

6. Système de transfusion selon l'une quelconque des revendications 1 à 5, dans lequel le dispositif de transfusion (10, 110) est une pompe péristaltique.

7. Procédé de commande d'un dispositif de commande (50) qui commande un dispositif de transfusion (10, 110), dans lequel
un moteur d'entraînement (25) qui entraîne une partie d'actionnement (12) compressant et relâchant séquentiellement un tube de transfusion (20, 120) du dispositif de transfusion (10, 110) est commandé, et
une détermination d'exactitude/inexactitude pour déterminer si un raccordement incorrect du tube de transfusion (20, 120) survient est réalisée sur la base d'informations d'arbre de moteur du moteur d'entraînement (25),
dans lequel les informations d'arbre de moteur comprennent une valeur de couple d'un arbre de moteur, le procédé étant **caractérisé en ce qu'**il comprend en outre
la génération d'une sous-trame des informations d'arbre de moteur dans une période au cours de laquelle un état d'entraînement du moteur d'entraînement (25) est dans un état prédéterminé, dans lequel l'état prédéterminé comprend qu'une vitesse de rotation de l'arbre de moteur du moteur d'entraînement (25) est supérieure ou égale à une valeur prédéterminée ;
l'utilisation de la sous-trame générée des informations d'arbre de moteur pour calculer, en tant qu'une valeur de caractéristique qui indique une caractéristique de l'état d'entraînement du moteur d'entraînement (25), une valeur moyenne d'un couple dans une section de la sous-trame ; et
la réalisation de la détermination d'exactitude/inexactitude du tube de transfusion (20, 120) sur la base de la valeur de caractéristique.

8. Programme informatique comprenant des instructions qui, lorsque le programme est exécuté par un ordinateur, amènent l'ordinateur à réaliser le procédé selon la revendication 7.
